# FASCICULE DE BREVET EUROPEEN

(11) **EP 1 003 472 B1**
(45) Date de publication et mention de la délivrance du brevet: **04.06.2003**
(21) Numéro de dépôt: 98941500.5
(22) Date de dépôt: 30.07.1998
(51) Int. Cl.: A61K 7/48

(54) **COMPOSITION PARFUMANTE UTILISANT DES ORGANOPOLYSILOXANES**
PARFÜMIERENDE ZUSAMMENSETZUNG MIT ORGANOPOLYSILOXANEN
PERFUME COMPOSITION USING ORGANOPOLYSILOXANES

(30) Priorité: 31.07.1997 FR 9709812; 09.04.1998 FR 9804483
(43) Date de publication de la demande: 31.05.2000
(73) Titulaire: RHODIA CHIMIE, 92408 Courbevoie Cédex (FR)
(72) Inventeur: BRANLARD, Paul, F-69005 Lyon (FR); MIGNANI, Gérard, F-69008 Lyon (FR); WILLEMIN, Claudie, F-75008 Paris (FR); OLIER, Philippe, F-69007 Lyon (FR)
(74) Mandataire: Wattremez, Catherine
(86) Numéro de dépôt international: FR9801699
(87) Numéro de publication internationale: WO99006017

(56) Documents cités:
- WO-A-92/16179
- WO-A-94/08557
- WO-A-95/31173
- US-A- 5 160 494
- US-A- 5 411 729

## Description

La présente invention a pour objet une composition parfumante comprenant une base parfumante et un agent solubilisant lipophile à base d'au moins un organopolysiloxane à fonction polaire ou polarisable ; elle a également pour objet l'utilisation des organopolysiloxanes à fonction polaire ou polarisable comme agents solubilisants des bases parfumantes et émollients dans les compositions parfumantes.

Un premier objet de l'invention consiste en une composition parfumante comprenant une base parfumante et un agent solubilisant lipophile de ladite base parfumante, ledit agent solubilisant étant à base de phényléthylheptaméthyltrisiloxane.

La composition parfumante faisant l'objet de l'invention peut comprendre de l'ordre de
- 3 à 20% de son poids d'une base parfumante et
- 75 à 97% de son poids d'un agent solubilisant lipophile à base dudit phényléthylheptaméthyltrisiloxane un des organopolysiloxanes à fonction polaire ou polarisable de formule (l) ou (l').

La base parfumante présente peut être tout composé utilisé dans l'industrie de la parfumerie et responsable des diverses notes parfumées. On distingue : parmi les notes fraiches hespéridées, les eaux de cologne et eaux fraîches ; parmi les notes florales, les types simple, fleurie, fleurie verte et fleurie aldéhydée ; parmi les notes fougères, la fougère ambrée ou aromatique ; parmi les notes orientales, les types épicée orientale et fleurie ambrée ; parmi les notes chyprées, les types chyprée fruitée, chyprée fleurie aldéhydée, chyprée verte, chyprée cuiré
A titre d'exemple de composés chimiques pouvant entrer dans la composition de ladite base parfumante, on peut citer à titre non limitatif l'acétophénone, la méthylacétophénone, les aldéhydes cinamique, amylcinnamique, lanisique, cuminique, cyclamen, hydratropic, le p-crésyl méthyl ether, la benzophénone, le citral, le citronellyl oxyacétaldéhyde, l'allyl hexanoate, l'amyl hexanoate, l'isobutyrate de cinnamyl, l'acétate ou phénylacétate de géranyle, les acétates de linalyle, menthyle, phényléthyle, vétivéryle, de jasmyle, le formate de phényléthyle, l'éthylméthylphényl glycidate, l'eugénol, l'isoeugénol, le géraniol, le citronetlal, l'hydroxycitronellal, l'ionone, la méthylionone, le phénylacétaldéhyde diméthylacétal, le menthol, les muscs, le phényléthyl alcool, les dérivés du pinène et du camphène, la carvone, le cinnamyl alcool, la coumarine, le diméthylbenzylcarbinyl acétate, l'héliotropine, l'isocyclocitral, le méthylnonyl acétaldéhyde, l'undécalactone, la vanilline ... pris seuls ou en mélanges entre eux.

La composition parfumante faisant l'objet de l'invention est une solution.

L'agent solubilisant présent peut être constitué du phényléthylheptaméthyltrisiloxane.

Selon une variante de réalisation, ledit agent solubilisant est constitué du phényléthylheptaméthyltrisiloxane associé à au moins un autre solvant volatil ou non volatil des bases parfumantes, comme les silicones volatils (par exemple hexaméthyldisiloxane, décaméthylcyclopentasiloxane, polydiméthylsiloxanes linéaires volatils , alkylpolyméthylsiloxanes volatils linéaires ou cycliques dont le radical alkyl contient de 2 à 13 atomes de carbone décrits dans US-A-5,160,494), l'éthanol, le propylène glycol, les esters (dioctanoate ou diisononanoate de diéthylène glycol par exemple).
Ledit ou lesdits solvants autres peuvent être présents selon un rapport pondéral organopolysiloxane(s) à fonction polaire ou polarisable de formule (I) ou (I') / solvant(s) autre(s) de l'ordre de 5/95 à 95/5, de préférence de l'ordre de 10/90 à 90/10, tout particulièrement de l'ordre de 25/75 à 90/10.

D'une manière toute préférentielle, ledit agent solubilisant est constitué :
. par du phényléthyl heptaméthyltrisiloxane (PEHMTS) ou
. par un mélange (M) constitué
   * pour plus de 70% massique, généralement pour au moins 75% massique de phényléthyl heptaméthyltrisiloxane de formule
      Me₃ Si -O- Si (Me) (Z¹) -O- Si Me₃ , où Z¹ représente la fonction -CH₂-CH₂-Ph
   * pour moins de 25% massique, généralement de 10 à 20 % massique, de trisiloxane de formule
      Me₃ Si -O- Si (Me) (Z²) -O- Si Me₃ , où Z² représente la fonction -CH (CH₃) -Ph
   * et moins de 5% massique, généralement de 0 à 2% massique de trisiloxane de formule
      Me₃ Si -O- Si (Me) (Z³) -O- Si Me₃ , où Z³ représente la fonction -CH=CH-Ph
      formules où Me représente un radical méthyl et Ph un radical phényl,
ledit phényléthyl heptaméthyltrisiloxane ou ledit mélange (M) pouvant être seul ou associé à un solvant silicone volatil, hexaméthyldisiloxane notamment, et/ou de l'éthanol, selon un rapport pondéral (PEHMTS) / hexaméthyldisiloxane et/ou éthanol, ou mélange (M) / hexaméthyldisiloxane et/ou éthanol, de l'ordre de 5/95 à 95/5, de préférence de l'ordre de 10/90 à 90/10, tout particulièrement de l'ordre de 25/75 à 90/10.
Cette association permet de préparer des compositions parfumantes à partir de bases parfumantes de polarités très diverses, notamment aussi diverses que celles de la coumarine, de la vanilline, de l'acétophénone et du lavandin, bases parfumantes présentant dans l'espace de solubilité de Hansen les paramètres suivants :

| | lavandin | acétophénone | vanilline | coumarine |
|---|---|---|---|---|
| .δ_{D} (J/cm³)^{½} | 17,1 | 19,6 | 16,6 | 19,25 |
| . δ_{P} (J/cm³)^{½} | 7,6 | 8,6 | 12,1 | 12,2 |
| .δ_{H} (J/cm³)^{½} | 7,6 | 3,7 | 12 | 6,8 |

Les organopolysiloxanes à fonction polaire ou polarisable de formule (I) ou (I') confèrent en outre à la composition parfumante faisant l'objet de l'invention, des propriétés sensorielles (notamment toucher sec sans résidu).

La composition parfumante faisant l'objet de l'invention, peut comprendre en outre jusqu'à 5% de son poids d'autres constituants, comme des agents conservateurs, des agents de stabilisation à la lumière, à l'oxygène, des colorants, des agents rafraîchissants tels que le mentyl lactate et le menthone glycerin acétal, des agents hydratants, des conservateurs ...

La composition parfumante faisant l'objet de l'invention se présente sous forme d'une solution ; elle peut être utilisée comme parfum sans alcool ou à faible quantité d'alcool (telle quelle ou sur un support textile ou autre) ou comme additif pour parfumer les formulations cosmétiques (crèmes de soins, déodorants, antiperspirants, produits de rasage ...).

Un deuxième objet de l'invention consiste en l'utilisation, dans les compositions parfumantes comprenant une base parfumante, des organopolysiloxanes à fonction polaire ou polarisable à base de phényléthylheptaméthyltrisiloxane comme agents solubilisants de ladite base parfumante.
La présente invention vise en outre l'utilisation, dans les compositions parfumantes comprenant une base parfumante, des organopolysiloxanes à fonction polaire ou polarisable à base de phényléthylheptaméthyltrisiloxane comme agents émollients (apportant des propriétés sensorielles).
Un troisième objet de l'invention consiste en un procédé de préparation d'une composition parfumante liquide contenant une base parfumante, par mise en solution de ladite base parfumante à l'aide d'au moins un des organopolysiloxanes à fonction polaire ou polarisable à base de phényléthylheptaméthyltrisiloxane.

La présente invention vise en outre un procédé pour apporter des propriétés sensorielles à une composition parfumante liquide contenant une base parfumante, par mise en solution de ladite base parfumante à l'aide d'au moins un des organopolysiloxanes à fonction polaire ou polarisable à base de phényléthylheptaméthyltrisiloxane

Les organopolysiloxanes à fonction polaire ou polarisable à base de phényléthylheptaméthyltrisiloxane, les quantités desdits organopolysiloxanes utilisées, ainsi que celles de base parfumante et des autres solvants ou additifs éventuellement présents, ont déjà été mentionnés ci-dessus.

Les exemples suivants sont donnés à titre non limitatif.

### Exemple 1

### Hydrosilylation du styrène par l'heptaméthyltrisiloxane

Dans un réacteur de 10 litres, on introduit à l'aide d'une pompe 1803 g (11,12 moles) d'hexaméthyldisiloxane (HMDS) et 4,15 g d'une solution de platine de Karstedt titrant 11,5 % de platine (0). La masse réactionnelle est portée à 90°C et on coule en 5 heures de façon simultanée 4150 g (19,3 moles) d'heptaméthyltrisiloxane (MD'M) et 2207 g (21,22 moles) de styrène.
Le suivi des espèces majoritaires par chromatographie en phase gazeuse montre que la réaction est pratiquement quantitative (en % poids).

| **durée** | **HMDS** | **styrène** | **MD'M** | **X-HMTS** | **Y-HMTS** | **Z-HMTS** |
|---|---|---|---|---|---|---|
| 1 h | 54,9 | 0,6 | 1,5 | 30,4 | 0,8 | 5,6 |
| 2h | 36,9 | 2,0 | 1,6 | 42,9 | 1,7 | 7,8 |
| 3h | 30,3 | 2,3 | 2,1 | 47,7 | 2,5 | 8,3 |
| 4h | 24,2 | 2,1 | 2,8 | 53,2 | 3,2 | 9,3 |
| 5h | 21,7 | 3,2 | 2,5 | 53,2 | 3,3 | 9,3 |

La teneur en styrène libre dans la masse réactionnelle, en fin de réaction, représente 88% de l'excès de styrène engagé, ce qui est la preuve d'une polymérisation très faible. Le complément à 100% est constitué par le produit de réaction des sous-produits du MD'M (MD'DM et MM' notamment) et du styrène.
Dans ce tableau,
* X-HMTS a la signification suivante
   Me₃ Si -O- Si (Me) (X) -O- Si Me₃ , où X représente la fonction -CH₂-CH₂-Ph
* Y-HMTS a la signification suivante
   Me₃ Si -O- Si (Me) (Y) -O- Si Me₃ , où Y représente la fonction -CH=CH-Ph
* Z-HMTS a la signification suivante
   Me₃ Si -O- Si (Me) (Z) -O- Si Me₃ , où Z représente la fonction -CH (CH₃) -Ph
avec Me représentant méthyle et Ph phényle.

### Distillation

La masse réactionnelle est alors concentrée (évaporation des volatils à 110°C sous 20 mbars pendant 7 heures). On recueille 5827 g d'un produit coloré de composition suivante (valeurs en % poids) :

| **HMDS** | **styrène** | **MD'M** | **X-HMTS** | **Y-HMTS** | **Z-HMTS** |
|---|---|---|---|---|---|
| 0,13 | 0,13 | 0,12 | 77,8 | 4,9 | 14,6 |

### Hydrogénation

Dans un réacteur autoclave de 1 litre , on charge 700g de ce produit coloré.
On introduit 14g (soit 2 % poids) d'un catalyseur platine sur noir titrant 2 % de Pt. La masse réactionnelle est portée à 100°C sous une pression de 20 bar d'hydrogène. Au bout de trois heures de réaction sous agitation , la masse réactionnelle est refroidie et ramenée sous pression atmosphérique. On obtient après filtration 692 g d'un produit incolore de composition suivante :

| **HMDS** | **styrène** | **MD'M** | **X-HMTS** | **Y-HMTS** | **Z-HMTS** |
|---|---|---|---|---|---|
| 0,13 | 0 | 0,1 | 80,75 | 1,10 | 14,3 |

Après distillation sur une colonne garnie d'anneaux Rashig, (hauteur = 40 cm), on recueille 563,3 g (rendement = 81,4 %) d'un mélange (M) de composition suivante :

| **HMDS** | **styrène** | **MD'M** | **X-HMTS** | **Y-HMTS** | **Z-HMTS** |
|---|---|---|---|---|---|
| 0 | 0 | 0 | 81,5 | 1,04 | 16,6 |

### Exemple 2

La base parfumante mise en oeuvre est le Lavandin, huile essentielle de la Société Firmenich, présentant les paramètres de solubilité suivants
. δ_{D} de 17,1 (J/cm³)^{½}
. δ_{P} de 7,6 (J/cm³)^{½}
. δ_{H} de 7,6 (J/cm³)^{½}.

L'agent solubilisant lipophile à base d'au moins un organotrisiloxane à fonction polaire mis en oeuvre est le mélange (M) préparé à l'exemple 1

### Composition parfumante

. Lavandin 7% en poids
. mélange (M) 93%
Cette composition (solution) limpide ("clear") est obtenue par simple mélange de ses deux constituants à température ambiante.

### Exemple 3

### Composition parfumante

. Lavandin 15% en poids
. mélange (M) de l'exemple 1 50%
. hexaméthyldisiloxane 35%
Cette composition (solution) limpide ("clear") est obtenue par simple mélange de ses trois constituants à température ambiante.

### Exemple 4

Des compositions parfumantes ont été préparées par simple mélange à température ambiante de lavandin selon les quantités données dans le tableau suivant et du complément à 100% en poids en un des organopolysiloxanes à fonction polaire ou polarisable suivants :
- dérivé de limonène et d'hydrogénoheptaméthyltrisiloxane (A)
- dérivé de dimère de l'α méthylstyrène et d'hydrogénoheptaméthyltrisiloxane (B)
- dérivé de l'allylmalonate de diéthyle et d'hydrogénoheptaméthyltrisiloxane (C)
- diphényl tétraméthyl disiloxane Ph Si (Me)₂ - O -Si (Me)₂ Ph (D)
- mélange (M)
et comparées à une composition parfumante comprenant un organopolysiloxane non polaire et non polarisable dérivé de l'octène et d'hydrogénoheptaméthyltrisiloxane (O).

| | | | | | | |
|---|---|---|---|---|---|---|
| lavandin % | 7,4 | 7 | 7 | 11 | 10 | 7 |
| organopolysiloxane | (A) | (B) | (C) | (D) | (M) | (O) |
| aspect de la solution | L^{*} | L^{*} | L^{*} | L^{*} | L^{*} | T^{**} |

| | | | | | | |
|---|---|---|---|---|---|---|
| * L = limpide ("clear") | | | | | | |
| ** T = trouble ("turbid") | | | | | | |

On constate que, contrairement aux autres organopolysiloxanes, l'organopolysiloxane (O) ne permet pas d'obtenir une composition parfumante limpide avec du lavandin.

## Revendications

1. Composition parfumante comprenant une base parfumante et un agent solubilisant lipophile de ladite base parfumante, ledit agent solubilisant étant à base de phényléthylheptaméthyltrisiloxane.

2. Composition selon la revendication 1, **caractérisée en ce que** ledit agent solubilisant est à base d'un mélange d'heptaméthyltrisiloxanes à fonctions polarisables constitué:
* pour plus de 70 % massique, généralement pour au moins 75 % massique, de phényléthylheptaméthyltrisiloxane de formule Me₃ Si -O- Si (Me) (Z¹ ) -O- Si Me₃ , où Z¹ représente la fonction -CH₂-CH₂-Ph
* pour moins de 25 % massique, généralement de 10 à 20 % massique, de trisiloxane de formule Me₃ Si -O- Si (Me) (Z²) -O- Si Me₃ , où Z² représente la fonction -CH (CH₃) -Ph
* et moins de 5 % massique, généralement de 0 à 2 % massique de trisiloxane de formule Me₃ Si -O- Si (Me) (Z³) -O- Si Me₃ , où Z³ représente la fonction -CH=CH-Ph
formules où Me représente un radical méthyle et Ph un radical phényle.

3. Composition selon l'une quelconque des revendications 1 ou 2 **caractérisée en ce qu'**elle comprend de l'ordre de :
- 3 à 20 % de son poids d'une base parfumante et
- 75 à 97 % de son poids d'un agent solubilisant à base de phényléthylheptaméthyltrisiloxane.

4. Composition selon l'une quelconque des revendications 1 à 3, **caractérisée en ce que** la base parfumante présente est choisi parmi tout composé utilisé dans l'industrie de la parfumerie et responsable des diverses notes parfumées.

5. Composition selon l'une quelconque des revendications précédentes, **caractérisée en ce que** l'agent solubilisant est constitué:
. par du phényléthylheptaméthyltrisiloxane (PEHMTS) ou
. par un mélange (M) constitué
* pour plus de 70 % massique, généralement pour au moins 75 % massique de phényléthylheptaméthyltrisiloxane de formule Me₃ Si -O- Si (Me) (Z¹) -O- Si Me₃ ,
où Z¹ représente la fonction -CH₂-CH₂-Ph
* pour moins de 25 % massique, généralement de 10 à 20 % massique, de trisiloxane de formule Me₃ Si -O- Si (Me) (Z²) -O- Si Me₃ , où Z² représente la fonction -CH (CH₃) -Ph
* et moins de 5% massique, généralement de 0 à 2% massique de trisiloxane de formule Me₃ Si -O- Si (Me) (Z³) -O- Si Me₃ , où Z³ représente la fonction -CH=CH-Ph
formules où Me représente un radical méthyl et Ph un radical phényl,
ledit (PEHMTS) ou ledit mélange (M) étant seul ou associé à au moins un autre solvant volatil ou non volatil des bases parfumantes, selon un rapport pondéral PEHMTS / autre solvant, ou mélange M / autre solvant, de l'ordre de 5/95 à 95/5, de préférence de l'ordre de 10/90 à 90/10, tout particulièrement de l'ordre de 25/75 à 90/10.

6. Composition selon la revendication 4, **caractérisée en ce que** l'autre solvant est un solvant silicone volatil, notamment l'hexaméthyldisiloxane et/ou l'éthanol.

7. Composition selon l'une quelconque des revendications précédentes, **caractérisée en ce qu'**elle ne comprend pas d'alcool.

8. Utilisation de la composition parfumante faisant l'objet de l'une quelconque des revendications 1 à 7, comme parfum éventuellement sans alcool, ou comme additif pour parfumer les formulations cosmétiques.

9. Utilisation, dans les compositions parfumantes comprenant une base parfumante, de phényléthylheptaméthyltrisiloxane, seul ou en mélange avec des solvants autres, comme agent solubilisant de ladite base parfumante.

10. Utilisation selon la revendication 9, **caractérisée en ce que** ledit phényléthylheptaméthyltrisiloxane est un agent émollient desdites compositions parfumantes.

11. Utilisation selon la revendication 9 ou 10, **caractérisée en ce que** ledit phényléthylheptaméthyltrisiloxane, seul ou en mélange avec des solvants autres, est mis en oeuvre à raison de 75 à 97 % du poids desdites compositions parfumantes renfermant de 3 à 20 % de leur poids de base parfumante.

12. Procédé de préparation d'une composition parfumante liquide contenant une base parfumante, par mise en solution de ladite base parfumante à l'aide de phényléthylheptaméthyltrisiloxane, seul ou ou en mélange avec des solvants autres.

13. Procédé selon la revendication 12, **caractérisé en ce que** ledit phényléthylheptaméthyltrisiloxane, seul ou ou en mélange avec des solvants autres, est mis en oeuvre à raison de 75 à 97 % du poids desdites compositions parfumantes renfermant de 3 à 20 % de leur poids de base parfumante.

## Patentansprüche

1. Parfümierende Zusammensetzung, die einen Parfümgrundstoff und ein lipophiles Lösemittel für besagten Parfümgrundstoff umfasst, wobei besagtes Lösemittel auf Phenylethylheptamethyltrisiloxan basiert.

2. Zusammensetzung gemäß Anspruch 1, **dadurch gekennzeichnet, dass** besagtes Lösemittel auf einem Gemisch von Heptamethyltrisiloxanen mit polarisierbaren Funktionen basiert, das besteht:
* für mehr als 70 Masse-%, im Allgemeinen für wenigstens 75 Masse-%, aus Phenylethylheptamethyltrisiloxan der Formel Me₃Si-O-Si(Me)(Z¹)-O-SiMe₃, worin Z¹ die Funktion -CH₂-CH₂-Ph darstellt,
* für weniger als 25 Masse-%, im Allgemeinen 10 bis 20 Masse-%, aus Trisiloxan der Formel Me₃Si-O-Si(Me)(Z²)-O-SiMe_{3,} worin Z² die Funktion -CH(CH₃)-Ph darstellt.
* und aus weniger als 5 Masse-%, im Allgemeinen 0 bis 2 Masse-% Trisiloxan der Formel Me₃Si-O-Si(Me)(Z³)-O-SiMe₃, worin Z³ die Funktion -CH=CH-Ph darstellt, Formeln, worin Me einen Methylrest und Ph einen Phenylrest darstellt.

3. Zusammensetzung gemäß einem der Ansprüche 1 oder 2, **dadurch gekennzeichnet, dass** sie umfasst in der Größenordnung von:
- 3 bis 20 % ihres Gewichts eines Parfümgrundstoffs und
- 75 bis 97 % ihres Gewichts eines Lösemittels auf der Basis von Phenylethylheptamethyltrisiloxan.

4. Zusammensetzung gemäß einem der Ansprüche 1 bis 3, **dadurch gekennzeichnet, dass** der vorliegende Parfümgrundstoff unter jeder Verbindung, die in der Parfümerieindustrie verwendet wird, ausgewählt und für die verschiedenen Duftnoten verantwortlich ist.

5. Zusammensetzung gemäß einem der vorhergehenden Ansprüche, **dadurch gekennzeichnet, dass** das Lösemittel besteht:
· aus Phenylethylheptamethyltrisiloxan (PEHMTS) oder
· aus einem Gemisch (M), das besteht:
* für mehr als 70 Masse-%, im Allgemeinen für wenigstens 75 Masse-%, aus Phenylethylheptamethyltrisiloxan der Formel Me₃Si-O-Si(Me)(Z¹)-O-SiMe₃, worin Z¹ die Funktion -CH₂-CH₂-Ph darstellt,
* für weniger als 25 Masse-%, im Allgemeinen 10 bis 20 Masse-%, aus Trisiloxan der Formel Me₃Si-O-Si(Me)(Z²)-O-SiMe₃, worin Z² die Funktion -CH(CH₃)-Ph darstellt,
* und aus weniger als 5 Masse-%, im Allgemeinen O bis 2 Masse-% Trisiloxan der Formel Me₃Si-O-Si(Me)(Z³)-O-SiMe₃, worin Z³ die Funktion -CH=CH-Ph darstellt,
Formeln, worin Me einen Methylrest und Ph einen Phenylrest darstellt,
wobei besagtes (PEHMTS) oder besagtes Gemisch (M) allein oder kombiniert mit wenigstens einem anderen flüchtigen oder nicht flüchtigen Lösungsmittel für die Parfümgnrndstoffe gemäß einem Gewichtsverhältnis PEHMTS / anderes Lösungsmittel oder Gemisch M / anderes Lösungsmittel in der Größenordnung von 5/95 bis 95/5, vorzugsweise in der Größenordnung von 10/90 bis 90/10, ganz speziell in der Größenordnung von 25/75 bis 90/10 ist

6. Zusammensetzung gemäß Anspruch 4, **dadurch gekennzeichnet, dass** das andere Lösungsmittel ein flüchtiges Siliconlösungsmittel, insbesondere Hexamethyldisiloxan und/oder Ethanol ist.

7. Zusammensetzung gemäß einem der vorhergehenden Ansprüche, **dadurch gekennzeichnet, dass** sie keinen Alkohol umfasst.

8. Verwendung der parfümierenden Zusammensetzung, die Gegenstand von einem der Ansprüche 1 bis 7 ist, als Parfüm gegebenenfalls ohne Alkohol oder als Zusatz zum Parfümieren der kosmetischen Formulierungen.

9. Verwendung in den parfümierenden Zusammensetzungen, die einen Parfümgrundstoff umfassen, von Phenylethylheptamethyltrisiloxan allein oder im Gemisch mit anderen Lösungsmitteln als Lösemittel für besagten Parfümgrundstoff.

10. Verwendung gemäß Anspruch 9, **dadurch gekennzeichnet, dass** besagtes Phenylethylheptamethyltrisiloxan ein Erweichungsmittel besagter parfümierender Zusammensetzungen ist.

11. Verwendung gemäß Anspruch 9 oder 10, **dadurch gekennzeichnet, dass** besagtes Phenylethylheptamethyltrisiloxan allein oder im Gemisch mit anderen Lösungsmitteln in einer Menge von 75 bis 97 % des Gewichts besagter parfümierender Zusammensetzungen, die 3 bis 20 % ihres Gewichts an Parfümgrundstoff enthalten, eingesetzt wird.

12. Verfahren zur Herstellung einer flüssigen parfümierenden Zusammensetzung, die einen Parfümgrundstoff enthält, durch Lösen besagten Parfümgrundstoffs mit Hilfe von Phenylethylheptamethyltrisiloxan allein oder im Gemisch mit anderen Lösungsmitteln.

13. Verfahren gemäß Anspruch 12, **dadurch gekennzeichnet, dass** besagtes Phenylethylheptamethyltrisiloxan allein oder im Gemisch mit anderen Lösungsmitteln in einer Menge von 75 bis 97 % des Gewichts besagter parfümierender Zusammensetzungen, die 3 bis 20 % ihres Gewichts an Parfümgrundstoff enthalten, eingesetzt wird.

## Claims

1. Fragrance composition comprising a fragrance base and a lipophilic agent for dissolving the said fragrance base, the said solubilizing agent being based on phenylethylheptamethyltrisiloxane.

2. Composition according to Claim 1, **characterized in that** the said solubilizing agent is based on a mixture of heptamethyltrisiloxanes containing polarizable functions consisting:
* for more than 70% by mass, generally for at least 75% by mass, of phenylethylheptamethyltrisiloxane of formula Me₃Si-O-Si(Me)(Z¹ )-O-SiMe₃ in which Z¹ represents the -CH₂-CH₂-Ph function
* for less than 25% by mass, generally from 10 to 20% by mass, of trisiloxane of formula Me₃Si-O-Si(Me) (Z² )-O-SiMe₃, in which Z² represents the -CH(CH₃)-Ph function
* and less than 5% by mass, generally from 0 to 2% by mass, of trisiloxane of formula Me₃Si-O-Si(Me) (Z³ )-O-SiMe₃, in which Z³ represents the -CH=CH-Ph function in which formulae Me represents a methyl radical and Ph represents a phenyl radical.

3. Composition according to either of Claims 1 and 2, **characterized in that** it comprises from about:
- 3 to 20% of its weight of a fragrance base, and
- 75 to 97% of its weight of a solubilizing agent based on phenylethylheptamethyltrisiloxane.

4. Composition according to any one of Claims 1 to 3, **characterized in that** the fragrance base present is chosen from any compound used in the perfume industry and responsible for various perfume notes.

5. Composition according to any one of the preceding claims, **characterized in that** the solubilizing agent consists:
• of phenylethylheptamethyltrisiloxane (PEHMTS) or
• of a mixture (M) consisting:
* for more than 70% by mass, generally for at least 75% by mass, of phenylethylheptamethyltrisiloxane of formula Me₃Si-O-Si (Me) (Z¹ )-O-SiMe₃, in which Z¹ represents the -CH₂-CH₂-Ph function
* for less than 25% by mass, generally from 10 to 20% by mass, of trisiloxane of formula Me₃Si-O-Si (Me) (Z²)-O-SiMe₃, in which Z² represents the - CH(CH₃)-Ph function
* and less than 5% by mass, generally from 0 to 2% by mass, of trisiloxane of formula Me₃Si-O-Si (Me) (Z³)-O-SiMe₃, in which Z³ represents the -CH=CH-Ph function
in which formulae Me represents a methyl radical and Ph represents a phenyl radical,
the said (PEHMTS) or the said mixture (M) being alone or combined with at least one other volatile or nonvolatile solvent for fragrance bases, in a PEHMTS/other solvent, or mixture M/other solvent, weight ratio from about 5/95 to 95/5, preferably from about 10/90 to 90/10, most particularly from about 25/75 to 90/10.

6. Composition according to Claim 4, **characterized in that** the other solvent is a volatile silicone solvent, in particular hexamethyldisiloxane, and/or ethanol.

7. Composition according to any one of the preceding claims, **characterized in that** it comprises no alcohol.

8. Use of the fragrance composition forming the subject of any one of Claims 1 to 7, as an optionally alcohol-free fragrance, or as an additive for fragrancing cosmetic formulations.

9. Use, in fragrance compositions comprising a fragrance base, of phenylethylheptamethyltrisiloxane, alone or as a mixture with other solvents, as an agent for dissolving the said fragrance base.

10. Use according to Claim 9, **characterized in that** the said phenylethylheptamethyltrisiloxane is an emollient for the said fragrance compositions.

11. Use according to Claim 9 or 10, **characterized in that** the said phenylethylheptamethyltrisiloxane, alone or as a mixture with other solvents, is used in a proportion of from 75 to 97% relative to the weight of the said fragrance compositions containing from 3 to 20% of their weight of fragrance base.

12. Process for preparing a liquid fragrance composition containing a fragrance base, by dissolving the said fragrance base using phenylethylheptamethyltrisiloxane, alone or as a mixture with other solvents.

13. Process according to Claim 12, **characterized in that** the said phenylethylheptamethyltrisiloxane, alone or as a mixture with other solvents, is used in a proportion of from 75 to 97% relative to the weight of the said fragrance compositions containing from 3 to 20% of their weight of fragrance base.
